# EUROPEAN PATENT SPECIFICATION

(11) **EP 0 633 029 B1**
(45) Date of publication and mention of the grant of the patent: **26.11.1997**
(21) Application number: 94113490.0
(22) Date of filing: 21.04.1989
(51) Int. Cl.: A61K 47/00

(54) **Further improvements relating to drug delivery systems**
Verbesserungen an Abgabesystemen für Arzneimittel
Amélioration aux systèmes de déliverance de médicaments

(30) Priority: 22.04.1988 GB 8809616
(43) Date of publication of application: 11.01.1995
(62) Divisional of application: 89905144.5
(73) Proprietor: CANCER RESEARCH CAMPAIGN TECHNOLOGY LIMITED, London NW1 4JL (GB); ZENECA LIMITED, London W1Y 6LN (GB)
(72) Inventor: Bagshawe, Kenneth D., CRC Lab. Dpt., Fulham Palace Road, London W6 8RF (GB); Rogers, Gordon T., CRC Lab. Dpt. of Medical Oncol., Fulham Palace Road, London W6 8RF (GB); Sharma, Surinder, CRC Lab. Dpt. of Medical Oncol., Fulham Palace Road, London W6 8RF (GB)
(74) Representative: Goldin, Douglas Michael

(56) References cited:
- BR. J. CANCER, vol.58, no.6 pages 700 - 703 BAGSHAWE K.D. ET AL. 'A CYTOTOXIC AGENT CAN BE GENERATED SELECTIVELY AT CANCER SITES.'

## Description

THIS INVENTION relates to systems for the control of neoplastic cell growth and is particularly concerned with systems involving the localisation of cytotoxic agents at tumour sites.

In our earlier Patent Application PCT/GB88/00181 (WO 88/07378) we disclose a two-component system which comprises
(i) a first component (Component A-E) that is an antibody fragment capable of binding with a tumour associated antigen, the antibody fragment being bound to an enzyme capable of converting a prodrug into a cytotoxic drug;
(ii) a second or final component (Component PD) that is a prodrug convertible under the influence of the enzyme to a cytotoxic drug (CD).

In our earlier PCT Patent Application and in this present Patent Application, the word 'tumour' is to be understood as referring to all forms of neoplastic cell growth including carcinomas, sarcomas, lymphomas and leukaemias.

The present invention provides a two component system which comprises:
i) a conjugate comprising
   (a) an antibody selected from antibody A5B7 and antibody SB10; linked to
   (b) an enzyme selected from carboxypeptidase G2 (CPG2) and nitroreductase; and
ii) a prodrug convertible under the influence of the conjugate into a cytotoxic drug.

The conjugate itself also forms part of the invention. A preferred conjugate comprises A5B7 antibody linked to CPG2 enzyme.

As described in our above-mentioned copending International Patent Application PCT/GB88/00181, the prodrug can be benzoic acid mustard glutamide that converts to benzoic acid mustard [p-(bis-2-choloroethyl)amino] benzoic acid under the influence of a carboxypeptidase. However, the principles of this invention are equally applicable to other prodrugs releasing benzoic acid mustard or analogues thereof or other cytotoxic drugs, in which the structural feature distinguishing the prodrug from the cytotoxic drug is removed by CPG2 or nitroreductase.

The antibody can be whole antibody or one of the antibody fragments, e.g. antibody lacking an Fc portion, F(ab')₂ or other fragment as described in our above-mentioned earlier filed International Patent Application. The function of the antibody is to assist in the localisation of the enzyme in the region of the tumour to be treated.

The antibody or fragment thereof may be conjugated directly, or indirectly through a linking component, to the enzyme. The conjugation can be effected by chemical bonding. Alternatively, a conjugate wherein the antibody is linked to the enzyme via a continuous polypeptide linkage may be made by splicing together nucleic acid sequences that code at least for one or more antigen binding sites and the enzyme and such other sequences as are necessary to retain the vector function of the molecule and the catalytic function of the enzyme when the gene product of the reconstructed nucleic acid sequence is expressed by eukaryotic or prokaryotic cells.

The antibody may be humanised. Reichmann L, Clark M, Waldmann H, and Winter G (Reshaping human antibodies for therapy - Nature 332: 323-327, 1988) shows that by genetic engineering techniques the antigen binding sites of a rodent monoclonal can be incorporated into human immunoglobulin fragments so that the immunogenicity of the molecule in the human subject is minimised. It has been shown that immunoglobulin-gene DNA can be manipulated so that the Fc portion of the antibody has been replaced with an active enzyme moiety (Neuberger MS, Williams GT, Fox RO - Nature 312: 604-608, 1984) and such genetically engineered constructs bearing one or more antigen binding sites and one or more enzyme active sites can be used in the present invention.

It has been observed that when monoclonal antibodies derived from one species are injected into another species the host antibody response may be (at least partially) directed at the idiotype of the injected monoclonal. (Rowe et al, IRCS Med Sci. 13: 936-7, 1985). Similarly, it is well-known that bacterial products, including enzymes, are immunogenic in mammalian species including man.

The present system will be most effective in man and suitable for repetitive use when the immunogenicity of the antibody-enzyme conjugate is minimised or if immune tolerance to such conjugates has been induced. This is likely to be achieved through genetic engineering methods since the production of monoclonals to specific antigens by human hybridomas has so far proved difficult to achieve consistently. It has been shown that the antigen binding site of a rodent monoclonal antibody can be incorporated into a human immunoglobulin framework (Reichmann et al, Nature 332: 323-327, 1988).

The immunogenicity of an enzyme of non-human origin may be reduced by modification of its amino acid sequence.

In order to render the antibody-enzyme conjugate less immunogenic, it can be modified by conjugation to polyethylene glycol or other polymers, e.g. by reaction with the cyanuric chloride derivative of methoxypolyethylene-glycol 5000. The resulting material may be employed directly, or may be pre-injected to render the host tolerant to further injections of the native conjugate. Reaction with synthetic copolymers of D-glutamine acid and d-lysine or with tri-peptidyl-modified organic polymers comprising alternate D-glutamic acid and D-lysine on the exterior ends of the side chains can be predicted to depress the immunogenicity of the conjugate. See, for example, Abuchowsky A., van Es T., Palezuk NC, Davis FF - J. Biol. Chem. 252: (11), 3578-81, 1977, or Kawamura K, Igarishji, T, Fujii T., Kamasaki J., Wada, H., Kishimoto, S. Int. Arch. Allergy appl. Immunol. 76: 324-330, 1985.

To minimise clinical problems arising from the use of immunogenic antibody enzyme conjugates, it is desirable to minimise or delay the production of host antibodies to xenospecific proteins by using immunosuppressive agents such as cyclosporin, cyclophosphamide, methotrexate, azathioprine etc., in order to provide sufficient time for the delivery of repeated treatments.

The ability of cyclosporin to prevent antimurine antibody responses by rabbits and in patients has been demonstrated. See, for example, Ledermann, JA. Begent, RHJ. Bagshawe, KD. Br. J. Cancer, 58: 562-566, 1988, or Ledermann, JA. Begent, RHJ. Riggs, SJ. Searle, F. Glaser, MG, Green, AJ. Dale, RG. Br. J. Cancer 58: 654-657, 1988.

In certain clinical conditions, it can be advantageous for the conjugate to be conjugated to a signal producing molecule such as a radioisotope suitable for scintigraphic imaging by gamma camera so as to confirm localisation of the conjugate at tumour sites.

Radiolabelling can be achieved with ¹²⁵I or ¹³¹I with standard methods either using chloramine T (Greenwood F, Hunter W, Glover JS, Biochem. J. 89: 114-123, 1963, Fraker PJ, Speck JC, Biochem. Biophys. Res. Comm. 80: 849-857, 1978), but other methods of iodination or radiolabelling with other isotopes such as indium or technetium can also be used. Such radiolabelled conjugates are generally used in clinical practice in amounts required for radioimmunolocalisation by immunoscintigraphy and would generally form only a small part of the administered conjugate.

Modern methods of analysis may be used in conjunction with a radiolabelled fraction of the conjugate to determine the concentration of the conjugate at target sites and non-target sites and thus help determine the optimum time for administration of the prodrug. (Riggs et al, Int. J. Cancer Supp. 2, 95-98, 1988, Dewhurst et al, (Abstract) Br. J. Cancer 1988).

The two components forming the system of the present invention are designed to be used in association with one another in a method of treatment of the human or animal body by therapy.

It is specifically designed for use in a method for the treatment of malignant diseases including carcinomas, sarcomas, lymphomas and leukaemias, which comprises administering to a host in need of such treatment an effective amount of a system.

In such a method, the conjugate is administered first and the prodrug is administered subsequent to the conjugate after a time interval such that the conjugate has selectively localised at the site of malignant cells.

The following Examples are given to illustrate various aspects of the invention.

### REFERENCE EXAMPLE 1

This is to illustrate the inactivation of CPG2 enzyme by an antibody. Such inactivation is useful for removing enzyme activity in circulation blood that has not localised to the tumour before administration of the prodrug, so that formation of active drug outside the tumour is limited.

A monoclonal antibody (SB43) was produced by conventional methods following immunisation of the lymphocyte donor mouse with carboxypeptidase G₂. Microtitre plates were coated with three units per well of carboxypeptidase G₂ and incubated with supernatants from the hybridoma culture, and it was found that ¹²⁵Iodine labelled rabbit anti-mouse antibody bound to the coated wells with a 50% binding titre at a dilution of the supernatant of 1:800 in buffered solution. Assay of enzyme activity was assessed after 24 hours incubation at 37°C in buffer containing a 1000-fold dilution of the antibody (hybridoma supernatant). Enzyme incubated with buffer alone for 24 hours retained most of its capacity to cleave methotrexate as shown in optical density measurements (54.1 carboxypeptidase units/ml initially falling to 40 units/ml activity after 24 hours). In the wells containing the antibody (hybridoma supernatant) the activity was reduced to 13.0 carboxypeptidase units/ml. The antibody alone had no effect on the optical density of methotrexate. These experiments show that the enzyme active site on the carboxypeptidase can be substantially inactivated by an antibody raised against the enzyme. Monoclonal antibodies to carboxypeptidase G₂, raised by the technique described above will only have a similar enzyme inhibiting property if they are directed at epitopes in or close to the active site of the enzyme.

### EXAMPLE 1

### Evidence for localisation of antibody-enzyme conjugate at tumour sites

1. 4 nude mice bearing LS174T human colon cancer xenografts on their L flanks were injected with A5B7 (Fab')₂ monoclonal antibody directed at carcinoembryonic antigen conjugated to carboxypeptidase G2 and labelled with ¹²⁵I. An immunoscintigraph taken after 48 hours confirms localisation of the conjugate at the tumour sites.
   Similar results were obtained using the following conjugates:
2. A5B7 intact IgG - carboxypeptidase
3. A5B7-F(ab')₂ - nitroreductase
4. SB10 (antiHCG) -F(ab')₂ - carboxypeptidase

### 2. METHODS OF CONJUGATION OF ANTIBODY TO ENZYME

Conjugation of IgG or F(ab')₂ with carboxypeptidase was accomplished by mixing a maleimide derivative of the enzyme with a thiolated antibody.

### 1) Thiolation with S-acetylthioglycolic acid N-hydroxysuccinimide ester (SATA).

IgG or F(ab')₂ in 0.1M sodium phosphate buffer, pH 7.6, (containing 372 mg of EDTA/litre) at 1-2 mg/ml was treated with a 15 molar excess of SATA (made up 20 mg/ml in DMF) and left at about 20^{o}C for approximately 2 hours. The thiolated antibody was then passed down a column of Sephadex G-25 (registered trade mark) to remove excess SATA. The thiol was deacetylated by adding 0.1 volumes of 3.5% hydroxylamine, pH 7.5, prepared by adding disodium hydrogen phosphate to an aqueous solution of hydroxylamine hydrochloride.

### 2) Thiolation with N-succinimidyl 3-(2-pyridyldithio)propionate (SPDP).

IgG or F(ab')₂ in 0.2M sodium phosphate buffer, pH 8.6, at a concentration of 4 mg/ml was treated with a 15 molar excess of SPDP in ethanol and left at r.t. for 1 hour. Excess SPDP was removed on a column of Sephadex G-25 (registered trade mark) equilibrated in 0.1M sodium acetate buffer, pH 4.5. The pyridyldisulphide group was then reduced for 30 minutes with 50 ul/ml of 100 mM dithiothreitol and excess reducing agent removed by Sephadex G-25 (registered trade mark) gel filtration.

### 3) Derivatisation of carboxypeptidase.

Carboxypeptidase in 0.1M sodium phosphate buffer, pH 7.6 (containing 372 mg EDTA/litre) at 2 mg/2.5 ml was treated for 3 hours with a 15 molar excess of succinimidyl 4-(p-maleimidophenyl)butyrate (SMPB) dissolved in THF. The excess SMPB was removed by gel filtration on Sephadex G-25 (registered trade mark).

### 4) Conjugation

The derivatised enzyme was mixed with an equimolar amount of thiolated antibody and the progress of the conjugation monitored by gel filtration. When no further reaction was judged to take place the mixture was concentrated and the conjugate purified by gel filtration. Typical enzyme activities obtained were 150-200 units/mg of conjugate.

### REFERENCE EXAMPLE 2

### Evidence that SB43 binds to carboxypeptidase G2 in vitro.

Microtitre wells were coated with carboxypeptidase G2 and monoclonal antibodies SB43 (raised to carboxypeptidase G2) and SB10 (raised to human chorionic gonadotrophin) were added in dilution and incubated before aspiration. ¹²⁵I anti-mouse IgG was then added and incubated for 30 minutes followed by aspiration and washing. The wells were cut out and counted in a gamma counter. The results set out below show no significant binding of SB10 to the carboxypeptidase coated wells but all dilutions of SB43 used showed high counts indicating binding to the carboxypeptidase. SB43 modified by addition of galactose moieties was included a similar dilution and showed similar binding to unmodified SB43.

The microtitre plate was coated with 0.1 microg. CPG₂ per well and incubated overnight with SB43. The ¹²⁵I-mouse IgG was then introduced, the plate incubated for 1 hour, washed and radio-counted.

| SAMPLE | TIME | COUNTS(1) | CPM(1) | %CV | |
|---|---|---|---|---|---|
| 1 | 30 | 1092 | 2171.4 | 3.1 | (Negative Control-SB10-Anti-hCG) |
| 2 | 30 | 805 | 1588.6 | 3.6 | " |
| 3 | 30 | 16428 | 33278.0 | .8 | SB43 x 20 |
| 4 | 30 | 23339 | 47302.9 | .7 | " |
| 5 | 30 | 22020 | 44679.3 | .7 | SB43 x 100 |
| 6 | 30 | 22096 | 44833.8 | .7 | " |
| 7 | 30 | 8437 | 16873.4 | 1.1 | SB43 x 1000 |
| 8 | 30 | 7671 | 15336.8 | 1.1 | " |
| 9 | 30 | 15411 | 31052.2 | .8 | SB43-Gal-10x20 |
| 10 | 30 | 15418 | 31066.3 | .8 | " |

### EXAMPLE 2

### Evidence that SB43 inactivates/clears Ab-E-conjugate in vivo from plasma

The level of carboxypeptidase G2 activity in plasma can be monitored by observing the hydrolytic cleavage of methotrexate, a folic acid analogue, to pteroates and L-glutamate. When a conjugate of A5B7-F(ab')₂-carboxypeptidase G2 (25 enzyme units) was injected intravenously and plasma samples obtained 20 hours later significant hydrolysis of methotrexate was observed equivalent to 1.12 to 1.45 enzyme units/ml as shown by the steps of the spectrophotometric print-out.

Mice which were injected with galactosylated anti-carboxypeptidase (SB43-Gal 10) 19 hours after A5B7F(ab')₂-CPG2 and plasma taken 5 minutes and 15 minutes later caused no significant hydrolysis of methotrexate showing that the enzyme had been inactivated and/or cleared from the plasma.

### EXAMPLE 3

### Biotinylation of antibody-enzyme conjugate

Carboxypeptidase G2 (44.4 mg) in 0.05M sodium bicarbonate buffer, pH 8.5 (1.5 ml) was mixed with sulphosuccinimidyl 1-6-(biotinamide) hexanoate (292 ug in 73 ul of buffer and left at room temperature for 3 hours. The enzyme was then separated on Sephadex G-25 (registered trade mark) equilibrated in 0.15M sodium phosphate buffer pH 7.6, containing 372 mg EDTA/litre and the volume adjusted to 2.5 ml. The biotinylated enzyme was then treated for 3 hours with a 15 molar excess of succinimidyl 4 - (p-maleimido phenyl)butyrate (SMPB) dissolved in tetrahydrofuran and the excess SMPB removed by gel filtration on Sephadex G25 (registered trade mark). The derivatised enzyme was then conjugated to thiolated F(ab')₂ fragment of the A5B7 antibody as described in Example 1.

Affinity purified avidin was used as obtained from Sigma Ltd., 10-15 units/mg protein. Mice received 20 ug of biotinylated A5B7-carboxypeptidase G2 conjugate followed after one hour by avid in in the dose range 20-500 ug. Rapid clearance of the enzyme activity in plasma was observed comparable to that observed with SB43 monoclonal antibody in Reference Example 2.

### EXAMPLE 4

IgG class immunoglobulins carrying different specificities on their two binding sites can be made by a fusion technique employing hybridomas producing different antibodies (Milstein C & Cuello AC. Nature 305: 537-540, 1983; Sfaerz UD & Bevan MJ. Proc. Nat. Acad. Sci. USA 83: 1453-1457, 1986) or by chemical conjugation of univalent preparations of each of the antibodies required as used here. F(ab')₂ fragments of monoclonals SB10 (anti-human chorionic gonadotrophin (anti-hCG)) and A5B7 (anti-carcinoembryonic antigen, (anti-CEA)) were reduced in the presence of arsenite. F(ab')₂ fragment (20 mg) in 0.1M sodium phosphate buffer pH 7.6 (10 ml) was mixed with sodium arsenite (12.4 mg) EDTA (3.72 mg) and 2-mercapto-ethylamine (1.13 mg) and left at room temperature. Solid 5,5'-dithio-bis-(2-nitrobenzoic acid) (19.8 mg) was added and the mixture left at about 20^{o}C for 18 hours. The thionitrobenzoate modified Fab' (TNB derivative) was purified by gel filtration on Sephadex G-25 (registered trade mark) with a yield of approximately 70% based on protein recovery.

The TNB derivative of anti-CEA (4.8 mg) in 5 ml of 0.1M sodium phosphate buffer, pH 6.8, containing 1mM EDTA was treated for 30 minutes with mercaptoethylamine to give a final concentration of 10mM. The reduced TNB-anti-CEA Fab' was then purified by gel filtration on Sephadex G-25 (registered trade mark) equilibrated in 0.1M sodium phosphate buffer pH 7.0, containing 1mM EDTA. The reduced TNB-anti-CEA Fab' was then incubated with 4.9 mg (5 ml) of TNB derivative of anti-hCG for 16 hours and the formation of bispecific antibody monitored by gel filtration on a Superose S-12 column (registered trade mark, Pharmacia). The yield was 20% based on protein after purification of the bispecific antibody on the Superose S-12 column (registered trade mark). The ability of this ¹²⁵I labelled biospecific antibody to function in vivo and bind to its corresponding antigen was demonstrated by injection into nude mice bearing either CEA producing LS174T tumours or hCG producing CC3 tumours. At 20 hours post-injection mean tumour to organ ratios were:

| anti CEA/ anti hCG | | non-specific F(ab')₂ |
|---|---|---|
| blood | 2.9 | 0.6 |
| liver | 3.9 | 1.9 |
| kidney | 1.8 | 1.2 |
| lung | 3.2 | 1.2 |
| spleen | 6 | 3.0 |
| colon | 9 | 5.3 |
| (Conjugation of A5B7 and SB43 (anticarboxypeptidase) has not yet been performed but above experiment demonstrates retention of binding site function). | | |

### EXAMPLE 5

### Method for galactosylation

Cyanomethyl 2,3,4,6-tetra-0-acetyl-1-thio-b-D) galactopyranoside (400 mg) in anhydrous methanol (10 ml) was treated with 5.4 mg of sodium methoxide in 1 ml of anhydrous methanol at about 20^{o}C for 48 hours. A stock solution of IgG in 0.25M sodium borate buffer, pH 8.5 at 1.3 mg/ml was prepared. Since the number of galactose residues conjugated to IgG was not determined, a unitage was adopted corresponding to the number of microlitres of the activated galactose derivative added to 200 ug of IgG at a concentration of 1.3 mg/ml.

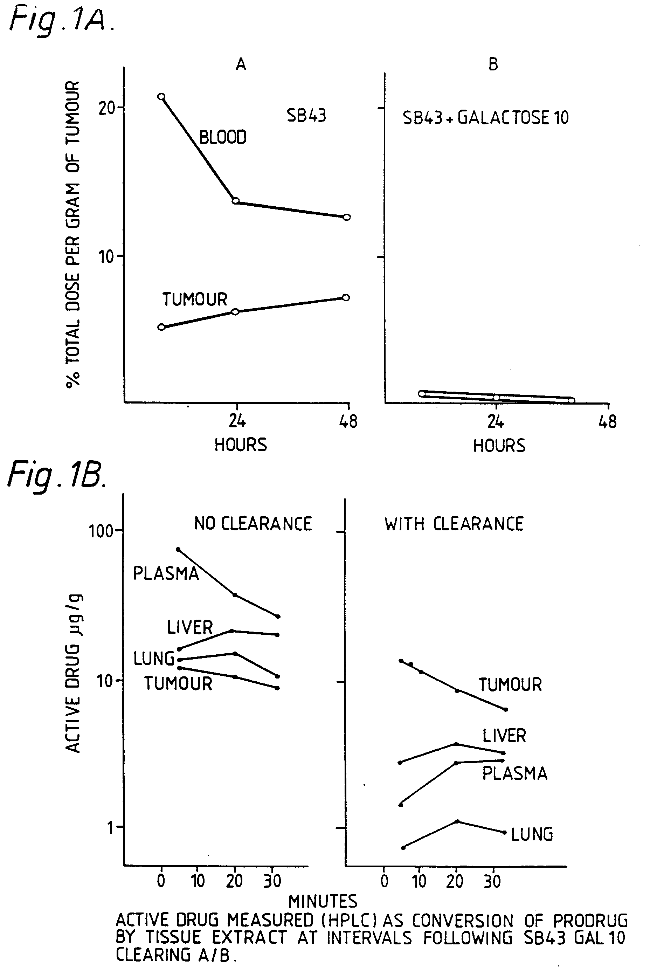

Aliquots of the activated galactose derivative (e.g. 300, 80, 40, 10, 5 and 2 ul) were dispensed into 3 ml glass ampoules and evaporated to a glassy residue in a stream of nitrogen under vacuum. 200 micrograms of IgG (153 ul of stock solution) were added to each aliquot and mixed until the residue was dissolved. After 2 hours at about 20^{o}C the solution was dialysed against 3 changes of PBS (phosphate buffer saline). Tests were performed to determine what level of galactosylation gave the most effective results. It was found that, in terms of the unitage defined above, 10 ul of the activated glactose derivative added to 200 ug of IgG gave the most satisfactory results. Figure 1A shows blood and tumour levels up to 48 hours after injection of monoclonal SB43 (anti-carboxypeptidase) A, native form;
B, galactosylated form. Further studies were performed, each in groups of 4 mice, bearing LS174T tumours and receiving A5B7 F(ab')2-CP (carboxypeptidase G2) conjugate followed after 24 hours by SB43 (anti-carboxypeptidase) galactosylated to the 10 ul level (as defined previously) or with saline as control followed one hour later by the bis-chloro benzoic acid mustard prodrug, 4-[bis-(2-chloroethyl)amino]-benzoic acid glutamide. Mice were killed at intervals following administration of the prodrug, the tissues extracted and prodrug and active drug levels were measured by HPLC methods.

Figure 1B shows the levels of active drug in various tissues in mice which had not received SB43-Gal 10 clearing antibody with those that had. In the absence of SB43-Gal 10 clearing antibody, levels of active drug were significantly lower than those found in liver and lung but in animals receiving the SB43-Gal 10 clearing antibody tumour levels were higher than in any other tissue.

As part of the same experiment two groups of mice, one with and one without SB43-Gal 10, were killed without receiving the prodrug. The tissues were extracted and tested for ability to convert prodrug to active drug in vitro, The results are shown in the Table and expressed as percentage of injected dose of carboxypeptidase per gram of tissue.

| In vivo admin | Carboxypeptidase G2% i.v. dose per gram of tissue at 48 hours | | |
|---|---|---|---|
| | Tumour | Plasma | T/P |
| Ab-CPG2 | 8.1(+0.69) | 0.22 | 36 |
| AB-CPG2 + Gal 10 antiCPG2 24 hours later | 7.2(+1.42) | 0.026 | 277 |

### EXAMPLE 6

Where the antigen corresponding to an intravenously administered antibody is present in the blood, antigen-antibody complexes form and these accelerate clearance of the antibody from the circulation into the reticuloendothelial cells. Accelerated clearance of anti-hCG antibodies W14 and SB10 occurs when these are injected into nude mice bearing CC3 hCG secreting tumours when compared with A5B7 anti-CEA antibody in LS174T bearing mice which express CEA on LS174T cell membranes but do not secrete CEA into the blood.

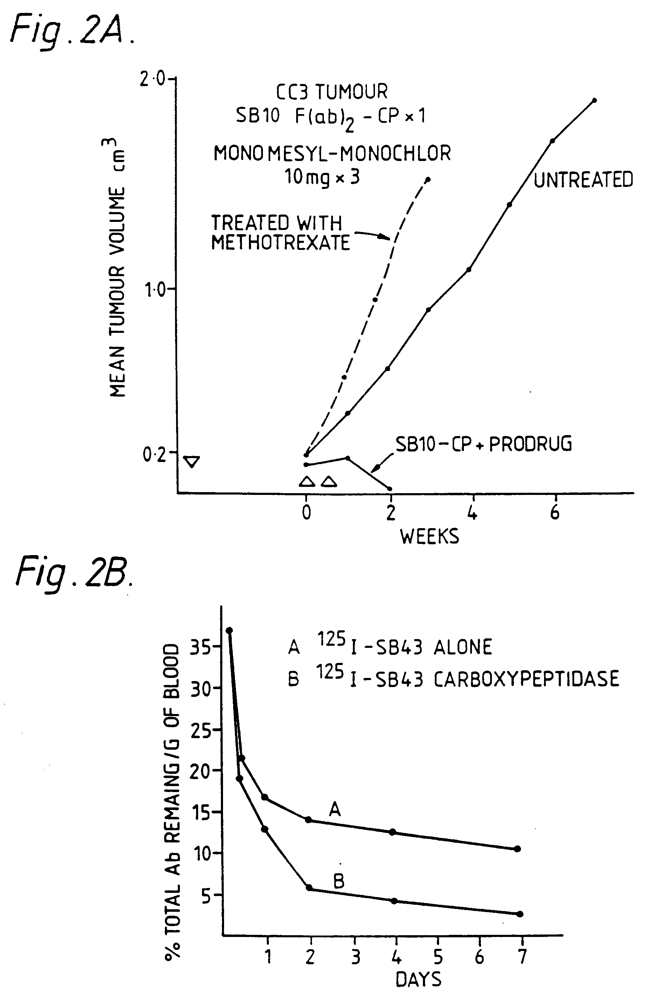

Figure 2A shows the result of administering SB10 F(ab')₂-CP (50 units CP) intravenously to 6 CC3 bearing nude mice, followed by the first of three injections (10 mg each) of the monomesyl monochloro benzoic acid mustard prodrug 4-[(2-chloroethyl)mesylamino]benzoic acid glutamide, the second given at 56 hours and the third at 72 hours. After 2 weeks the tumour was no longer detectable and the mice remain tumour free at 12 weeks. The growth of CC3 tumours in 6 untreated mice is also shown.

Attempts to introduce the prodrug into LS174T bearing mice before 120 hours after administration of A5B7 F(ab')₂-CP 50 units resulted in death of the animals and this was shown to be due to persisting enzyme activity in the blood.

Figure 2b shows accelerated clearance of 20 ug monoclonal ¹²⁵I-SB43 anticarboxypeptidase from the blood of A2G mice when 77 ug of the corresponding antigen, carboxypeptidase G2, was administered 1 hour later compared with controls which did not receive the antigen.

These data indicate that accelerated clearance of an administered antibody can be achieved by administration of a substance expressing the epitope corresponding to the binding site of the antibody.

### REFERENCE EXAMPLE 3

### Conjugation of TCK9 human albumin microspheres to SB43

1 mg of TCK9 human polyalbumin microspheres were derivatised with a 12.5M excess of sulpho-MBS (based on monomeric unit of 66 Kd) in a total of 1 ml phosphate buffer pH 7.8 for 2 hours at about 20^{o}C. The mixture was centrifuged at 3000 rpm for 3 minutes and resuspended in 1 ml of buffer, and rewashed once more. 1.5 mg of ¹²⁵I labelled SB43 was thiolated by 20M excess of SPDP, according to manufacturers (Pharmacia) instructions, at about 20^{o}C. The derivatised microspheres were centrifuged at 3000 rpm for 3 minutes, and then resuspended in the thiolated SB43 solution, the conjugation was carried out by incubating the mixture at 4^{o}C for 72 hours.

The antibody polyalbumin conjugate was separated from the reaction mixture by centrifuging at 13000 rpm (MSE Micro centaur) for 2 minutes; the pellet was resuspended in 250 ul of sterile saline for use.

### EXAMPLE 7

Asialofetuin was given intravenously at time zero and again at 120 minutes to mice bearing LS174T xenografts. ¹²⁵I-A5B7-galactosylated to 10 units was administered at time +5 minutes. Mice were killed at intervals and tissues excised and radioactivity levels counted. At 24 hours the tumour to blood ratio was 27.8:1 and the tumour to liver ratio 4.2:1. All other tissues showed even more favourable ratios. It should be recognised that whereas enzyme taken up by liver is rapidly inactivated, radioactivity persists in the organ.

## Claims

1. A conjugate comprising:
(a) an antibody selected from antibody A5B7 and antibody SB10; linked to
(b) an enzyme selected from carboxypeptidase G2 (CPG2) and nitroreductase.

2. A conjugate as claimed in claim 1 which comprises A5B7 antibody linked to carboxypeptidase G2 enzyme.

3. A conjugate according to claim 1 or claim 2 wherein the antibody lacks an Fc portion.

4. A conjugate according to any one of claims 1 to 3 wherein the antibody is a F(ab')₂ structure.

5. A conjugate according to any one of claims 1 to 4 wherein the antibody is linked to the enzyme via a continuous polypeptide linkage.

6. A conjugate according to any one of claims 1 to 5 wherein the antibody is humanised.

7. A conjugate according to claim 4 obtainable by mixing a maleimide derivative of the enzyme with thiolated antibody A5B7 F(ab')₂.

8. A conjugate according to claim 4 obtainable by:
(i) thiolation of antibody A5B7 F(ab')₂ with S-acetylthioglycolic acid N-hydroxysuccinimide ester (SATA);
(ii) derivatisation of carboxypeptidase G2 with succinimidyl 4-(p-maleimidophenyl)butyrate (SMPB);
(iii) mixing together the products of (i) and (ii) to form a conjugate; and
(iv) optionally purifying the conjugate.

9. A two component system which comprises:
i) a conjugate as defined in any one of claims 1-8; and
ii) a prodrug convertible under the influence of the conjugate into a cytotoxic drug.

10. A two component system which comprises:
i) a conjugate as defined in any one of claims 1-8; and
ii) a mustard prodrug convertible under the influence of the conjugate into a cytotoxic drug.

11. A conjugate as defined in any one of claims 1-8 for use in a method for the treatment of the human or animal body by therapy.

12. A conjugate as defined in claim 11 for use in a method for the treatment of a malignant disease.

13. A pharmaceutical composition comprising a conjugate as defined in any one of claims 1-8.

## Patentansprüche

1. Konjugat, umfassend:
(a) einen Antikörper, ausgewählt aus dem Antikörper A5B7 und dem Antikörper SB10; gebunden an
(b) ein Enzym, ausgewählt aus Carboxypeptidase G2 (CPG2) und Nitroreduktase.

2. Konjugat nach Anspruch 1, umfassend den A5B7-Antikörper, gebunden an das Carboxypeptidase-G2-Enzym.

3. Konjugat nach Anspruch 1 oder Anspruch 2, dadurch **gekennzeichnet,** daß dem Antikörper ein Fc-Teil fehlt.

4. Konjugat nach einem der Ansprüche 1 bis 3, dadurch **gekennzeichnet,** daß der Antikörper eine F(ab')₂-Struktur hat.

5. Konjugat nach einem der Ansprüche 1 bis 4, dadurch **gekennzeichnet,** daß der Antikörper an das Enzym über eine ununterbrochene Peptidbindung gebunden ist.

6. Konjugat nach einem der Ansprüche 1 bis 5, dadurch **gekennzeichnet,** daß der Antikörper humanisiert ist.

7. Konjugat nach Anspruch 4, erhältlich durch Mischen eines Maleimidderivats des Enzyms mit thioliertem Antikörper A5B7-F(ab')₂.

8. Konjugat nach Anspruch 4, erhältlich durch:
(i) Thiolierung des Antikörpers A5B7-F(ab')₂ mit S-Acetylthioglykolsäure-N-hydroxysuccinimidester (SATA);
(ii) Derivatisierung der Carboxypeptidase G2 mit Succinimidyl-4-(p-maleimidophenyl)butyrat (SMPB);
(iii) Zusammenmischen der Produkte von (i) und (ii), um ein Konjugat zu bilden; und
(iv) gegebenenfalls Reinigen des Konjugats.

9. Zweikomponentensystem, umfassend:
i) ein Konjugat, wie in einem der Ansprüche 1 bis 8 definiert; und
ii) ein Prodrug, das unter dem Einfluß des Konjugats in ein cytotoxisches Medikament umwandelbar ist.

10. Zweikomponentensystem, umfassend:
i) ein Konjugat, wie in einem der Ansprüche 1 bis 8 definiert; und
ii) ein N-Lost-Prodrug, welches unter dem Einfluß des Konjugats in ein cytotoxisches Medikament umwandelbar ist.

11. Konjugat, wie in einem der Ansprüche 1 bis 8 definiert, zur Verwendung in einem Verfahren zur Behandlung des menschlichen oder tierischen Körpers mittels Therapie.

12. Konjugat wie in Anspruch 11 definiert, zur Verwendung in einem Verfahren zur Behandlung einer malignen Erkrankung.

13. Pharmazeutisches Mittel, umfassend ein wie in einem der Ansprüche 1 bis 8 definiertes Konjugat.

## Revendications

1. Conjugué comprenant :
(a) un anticorps choisi parmi un anticorps A5B7 et un anticorps SB10 ; lié à
(b) une enzyme choisie parmi une carboxypeptidase G2 (CPG2) et une nitroréductase.

2. Conjugué tel que revendiqué à la revendication 1 qui comprend un anticorps A5B7 lié à une enzyme carboxypeptidase G2.

3. Conjugué selon la revendication 1 ou la revendication 2 dans lequel l'anticorps est dépourvu d'une partie Fc.

4. Conjugué selon l'une quelconque des revendications 1 à 3 dans lequel l'anticorps a une structure F(ab')₂.

5. Conjugué selon l'une quelconque des revendications 1 à 4 dans lequel l'anticorps est lié à l'enzyme par l'intermédiaire d'une liaison polypeptidique continue.

6. Conjugué selon l'une quelconque des revendications 1 à 5 dans lequel l'anticorps est humanisé.

7. Conjugué selon la revendication 4 pouvant être obtenu en mélangeant un dérivé maléimide de l'enzyme avec un anticorps A5B7 F(ab')₂ thiolé.

8. Conjugué selon la revendication 4 pouvant être obtenu par les étapes consistant :
(i) à thioler un anticorps A5B7 F(ab')₂ avec un ester de N-hydroxysuccinimide de l'acide S-acétylthioglycolique (SATA) ;
(ii) à faire un dérivé de carboxypeptidase G2 avec du 4-(p-maléimidophényl) butyrate de succinimidyle (SMPB) ;
(iii)à mélanger ensemble les produits de (i) et (ii) pour former un conjugué; et
(iv) éventuellement à purifier le conjugué.

9. Système à deux composants qui comprend :
(i) un conjugué tel que défini dans l'une quelconque des revendications 1-8; et
(ii) un promédicament convertible sous l'influence du conjugué en un médicament cytotoxique.

10. Système à deux composants qui comprend :
(i) un conjugué tel que défini dans l'une quelconque des revendications 1-8; et
(ii) un promédicament de type moutarde convertible sous l'influence du conjugué en un médicament cytotoxique.

11. Conjugué tel que défini dans l'une quelconque des revendications 1-8 destiné à être utilisé dans une méthode pour le traitement thérapeutique du corps humain ou animal.

12. Conjugué tel que défini dans la revendication 11 destiné à être utilisé dans une méthode pour le traitement d'une maladie maligne.

13. Composition pharmaceutique comprenant un conjugué tel que défini dans l'une quelconque des revendications 1-8.
